# EUROPEAN PATENT APPLICATION

(11) **EP 4 831 175 A1**
(43) Date of publication of application: **28.10.2026**
(21) Application number: 24905910.6
(22) Date of filing: 08.11.2024
(51) Int. Cl.: C07D 487/04, A61K 31/519, A61P 35/00, A61P 35/02

(54) **COMPOUND AND SALT THEREOF, USE IN PREPARATION OF DRUG FOR TREATING CANCER AND KINASE INHIBITOR, AND DRUG FOR TREATING CANCER**

(30) Priority: 19.12.2023 CN 202311747732
(71) Applicant: Pharmamax Pharmaceuticals, Ltd., Taizhou, Jiangsu 225300 (CN)
(72) Inventor: FAN, Fangtian, Taizhou, Jiangsu 225300 (CN); HE, Qiwei, Taizhou, Jiangsu 225300 (CN); TAO, Yu, Taizhou, Jiangsu 225300 (CN); DAI, Wenting, Taizhou, Jiangsu 225300 (CN)
(74) Representative: Osha BWB
(86) International application number: PCT/CN2024/130748
(87) International publication number: WO 2025/130423

(57) **Abstract**

The present invention belongs to the technical field of drug synthesis, and particularly relates to a compound and a salt thereof, the use in the preparation of a drug for treating cancer and a kinase inhibitor, and a drug for treating cancer. Provided in the present invention are a CHMFL-FLT3-122 compound deuterated at a specific position and a pharmaceutically acceptable salt thereof. Provided is the use of the compound or the pharmaceutically acceptable salt thereof in the preparation of a drug for treating cancer and a kinase inhibitor. Also provided is a drug for treating cancer. The compound and the pharmaceutically acceptable salt thereof provided by the present invention have a strong anti-cancer activity, good metabolic stability and pharmacokinetic properties, and have excellent application prospects.

## Description

### TECHNICAL FIELD

The present invention belongs to the technical field of pharmaceutical synthesis, and relates to a compound and a salt thereof, use in the preparation of a drug for treating cancer and a kinase inhibitor, and a drug for treating cancer.

### BACKGROUND

Blood cells in the blood or tissues are derived from hematopoietic stem cells. Hematopoietic stem cells undergo stepwise differentiation to produce various mature blood cells, a process known as hematopoiesis. Only when the number of mature blood cells and platelets is maintained at a certain level can the physiological functions of the body operate normally. When blood cell differentiation is hindered or proliferation is uncontrolled, the production of mature blood cells is insufficient, and a large number of immature blood cells are produced, occupying the space for normal blood cells, competing for nutrients, and causing bodily dysfunction. This is the occurrence of leukemia. Patients with leukemia are prone to persistent bleeding and decreased immunity, seriously endangering a patient's life.

Leukemia can be divided into four main types, namely Acute Myeloid Leukemia (AML), Chronic Myeloid Leukemia (CML), Acute Lymphocytic Leukemia (ALL), and Chronic Lymphocytic Leukemia (CLL). AML is the most common type of leukemia and also the type with the highest mortality rate. With the continuous development of gene sequencing technology, researchers have found that an increasing number of gene mutations play an important role in the pathogenesis of AML. Among them, mutations in the FLT3 kinase are the most common in AML, and patients with FLT3 gene mutations often have a poor prognosis.

The FLT3 kinase structure includes an extracellular domain; a transmembrane domain; a juxtamembrane domain; two kinase domains separated by an intermediate insertion structure; and finally a C-terminal domain. Under normal physiological conditions, in the absence of FLT3 ligand (FL), the juxtamembrane structure of FLT3 inhibits the dimerization of FLT3. When FL is present, it binds to the extracellular receptor domain, causing FLT3 dimerization, leading to structural changes in the kinase domain and phosphorylation of downstream signaling proteins such as PI3K, Ras, and STATS, thereby activating downstream signaling pathways and regulating cell growth, proliferation, etc. through a series of signal transductions. In 1996, when testing AML patients, researchers found that up to 30% of patient samples tested positive for the FLT3-ITD (FLT3 kinase juxtamembrane domain internal tandem duplication) mutation. Subsequently, in 2001, it was found in a research that 5% of patients had the FLT3-TKD point mutation (tyrosine kinase domain point mutation). Both mutations enable FLT3 kinase activation independently of FL binding. When the FLT3-ITD mutation is present, it causes non-FL dependent activation and activates downstream signaling pathways PI3K, Ras, and STATS, promoting cell proliferation and leading to the occurrence of AML.

The marketed first-generation FLT-3 inhibitors are all multi-target inhibitors, represented by Sorafenib (2005), Sunitinib (2006), Ponatinib (2013), and Cabozantinib (2013). Because the first-generation drugs are all multi-target drugs, they have some unavoidable side effects, such as diarrhea, anorexia, fatigue, nausea, rash, acne, arthralgia, etc. Consequently, more efficient FLT-3 inhibitors have been successively developed and approved to be on the market, with indications primarily focused on acute myeloid leukemia. Midostaurin received FDA breakthrough therapy designation, becoming the first FLT3-targeting kinase inhibitor. Quizartinib, as a second-generation FLT3 inhibitor, shows good selectivity and inhibitory activity for FLT3. The FLT3 kinase inhibitors currently developed have achieved good therapeutic effects in clinical trials. However, as FLT3 inhibitors are continuously advanced in clinical testing, the issue of drug resistance has rapidly emerged. For FLT3-ITD positive AML, there is still an unmet medical need.

Domestically, based on the structure of the recently reported BTK kinase inhibitor Ibrutinib, structural modifications were made to obtain the compound CHMFL-FLT3-122 (designated as D18), which has high inhibitory activity against FLT3-ITD positive AML cell lines. In the cellular environment, it affects FLT3-ITD-mediated signaling pathways and exhibits selectivity between BTK kinase and FLT3 kinase. It has currently entered Phase II clinical trials and is a potential drug candidate for treating FLT3-ITD positive AML , but its eventual successful market launch remains uncertain.

In summary, for patients with FLT3-ITD positive AML, providing compounds with superior performance and richer options with high inhibitory activity has very important practical significance.

### SUMMARY

The present invention provides a deuterated CHMFL-FLT3-122 compound and a pharmaceutically acceptable salt thereof, provides the use of the above compound or pharmaceutically acceptable salt thereof in the preparation of a drug for treating cancer and a kinase inhibitor, and also provides a drug for treating cancer, aiming to enhance drug efficacy and/or improve drug safety.

The present invention provides a compound or a pharmaceutically acceptable salt thereof, having the following structure:

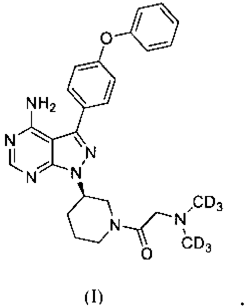

The present invention also provides another compound or a pharmaceutically acceptable salt thereof, having the following structure:

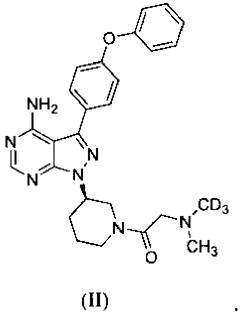

Further, the pharmaceutically acceptable salt is a salt formed from the compound of the present invention with an acid.

Further, the pharmaceutically acceptable salt is selected from one or more of phosphate, camphorsulfonate, hydrochloride, hydrobromide, hydrofluoride, sulfate, nitrate, formate, acetate, propionate, oxalate, malonate, succinate, fumarate, maleate, lactate, malate, tartrate, citrate, picrate, methanesulfonate, trifluoromethanesulfonate, benzenemethanesulfonate, and benzene sulfonate.

Preferably, the pharmaceutically acceptable salt is hydrochloride.

The present invention also provides a use of the above compound or the pharmaceutically acceptable salt thereof in the preparation of a drug for treating cancer.

Further, the use is a use of the above compound or the pharmaceutically acceptable salt thereof as a novel FLT3 kinase tyrosine kinase inhibitor to reduce or inhibit mutant FLT3 kinase activity in a cell or subject, and to prevent or treat a cell proliferative disorder and/or an FLT3-related disorder in a subject.

Further, the cancer is selected from lung cancer, colorectal cancer, pancreatic cancer, ovarian cancer, liver cancer, glioblastoma, solid tumors, non-small cell lung cancer, papillary renal cell carcinoma, and melanoma.

The present invention also provides a use of the above compound or the pharmaceutically acceptable salt thereof in the preparation of an FLT3 kinase inhibitor.

The present invention also provides a drug for treating cancer, wherein the drug is a formulation prepared using the above compound or the pharmaceutically acceptable salt thereof as an active ingredient, together with pharmaceutically acceptable adjuvants.

### Beneficial Effects

The compound and the pharmaceutically acceptable salt thereof provided by the present invention have strong anti-cancer activity.

The compound and the pharmaceutically acceptable salt thereof provided by the present invention also have good metabolic stability and pharmacokinetic properties.

### BRIEF DESCRIPTION OF DRAWINGS

FIG. 1 shows a cell cycle distribution of MOLM-13 cells under the action of compounds D18 and D18-D6.
FIG. 2 shows a cell cycle distribution of MV4-11 cells under the action of compounds D18 and D18-D6.
FIG. 3 shows colonies of MOLM-13 and MV4-11 cells under the action of compounds D18 and D18-D6.
FIG. 4A shows the change in mouse body weight after administration; FIG. 4B shows the change in tumor size in mice after administration; FIG. 4C shows actual photos of final tumors in mice after administration; and FIG. 4D shows the weight of final tumors in mice after administration.

### DETAILED DESCRIPTION

The spatial shape and volume of deuterium are essentially the same as hydrogen. When hydrogen in a drug molecular structure is replaced by deuterium, the biological activity and selectivity of the original drug are usually retained in the deuterated drug. The resulting deuterated drug exhibits isotope effects that modulate processes related to drug metabolism. However, due to the complexity of metabolic processes in biological systems, the pharmacokinetic properties of drugs in vivo also exhibit corresponding complexity due to the influence of multiple factors. Therefore, compared to non-deuterated drugs, changes in the pharmacokinetic properties of deuterated drugs show great contingency and unpredictability. For example, deuteration at certain sites may not extend the half-life but may instead shorten it (Scott L. Harbeson, Roger D. Tung. Deuterium in Drug Discovery and Development, P405-406).

Based on the CHMFL-FLT3-122 (designated as D18) compound, the present invention prepared a series of deuterated products, and from these, unexpectedly discovered two new compounds that have significant inhibitory effects on both human acute monocytic leukemia cell strain MV4-11 and human acute myeloid leukemia cell strain MOLM-13.

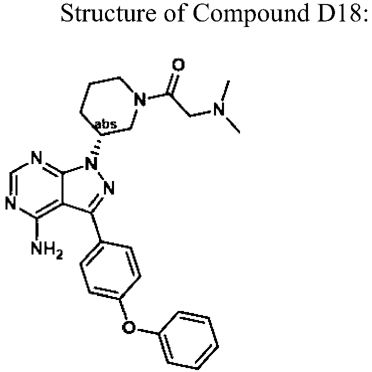

The series of compounds tested in the present invention includes Compounds 2-15. The structures thereof are as follows.

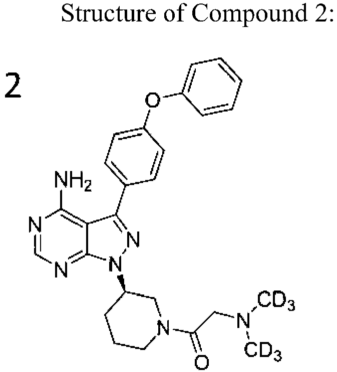

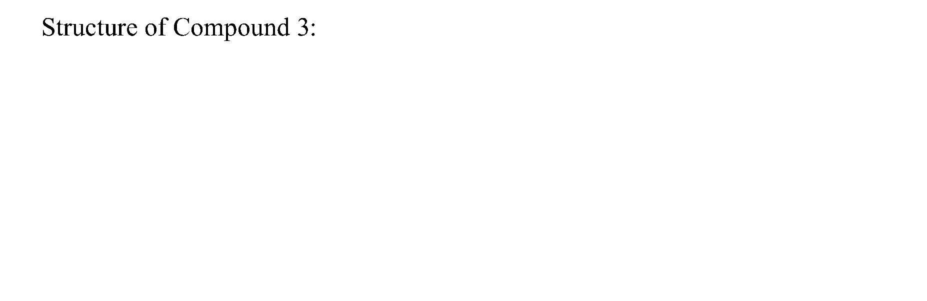

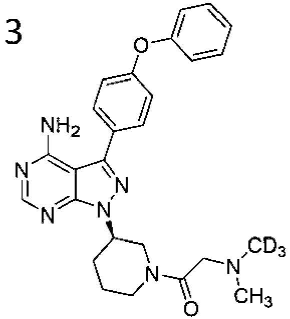

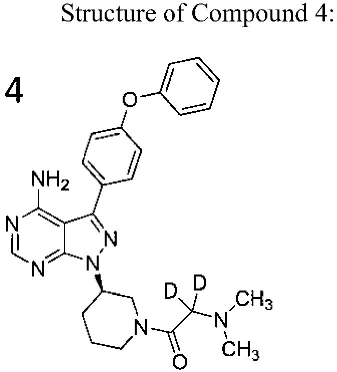

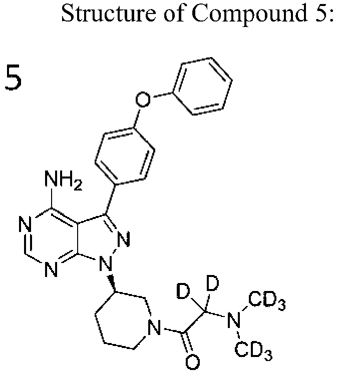

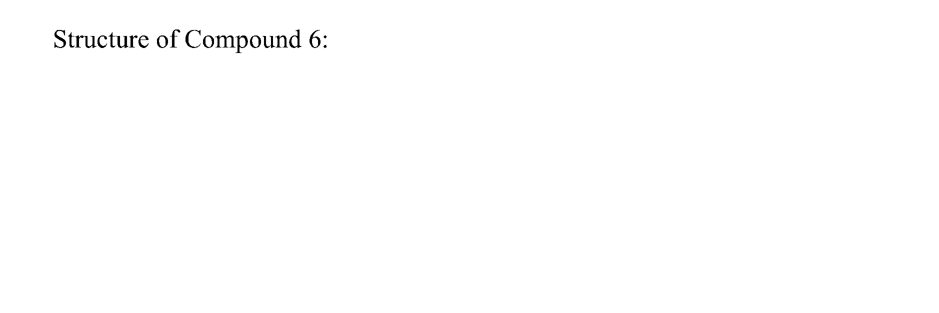

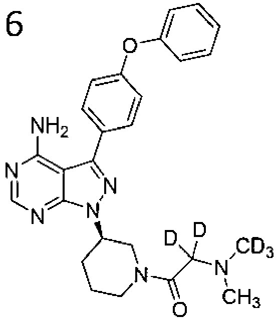

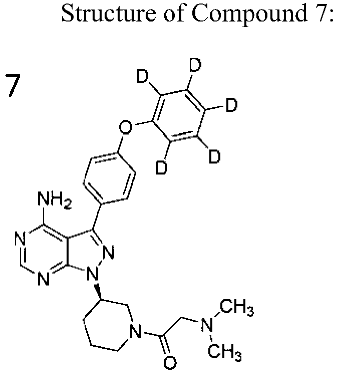

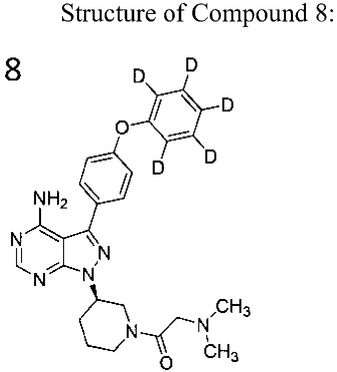

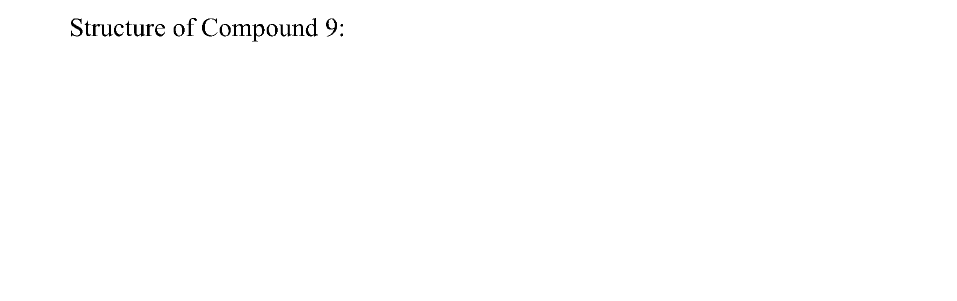

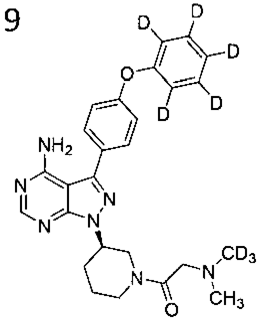

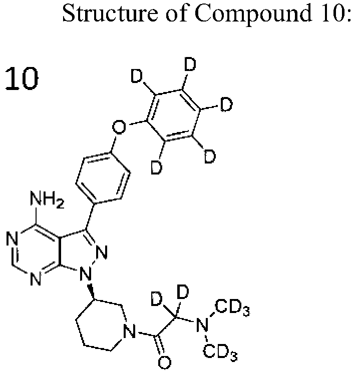

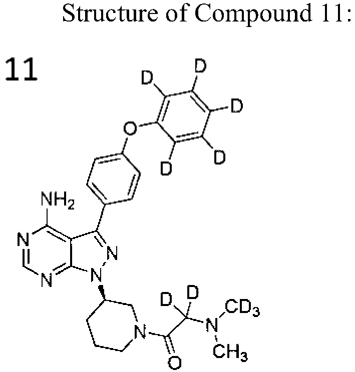

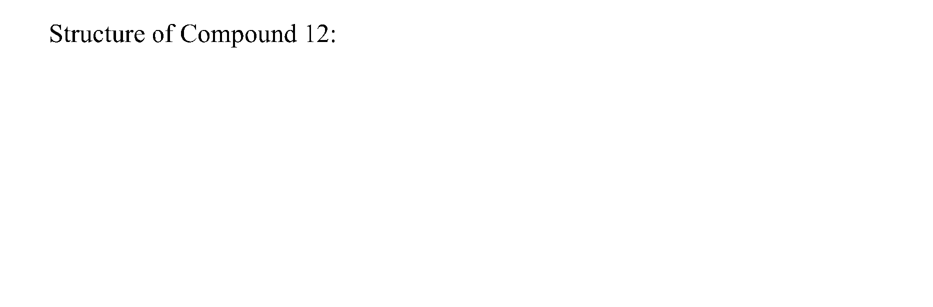

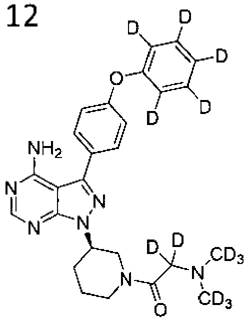

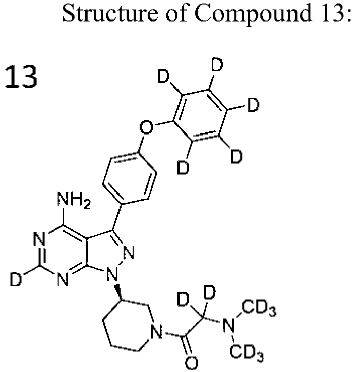

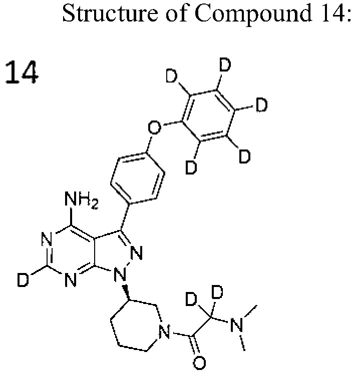

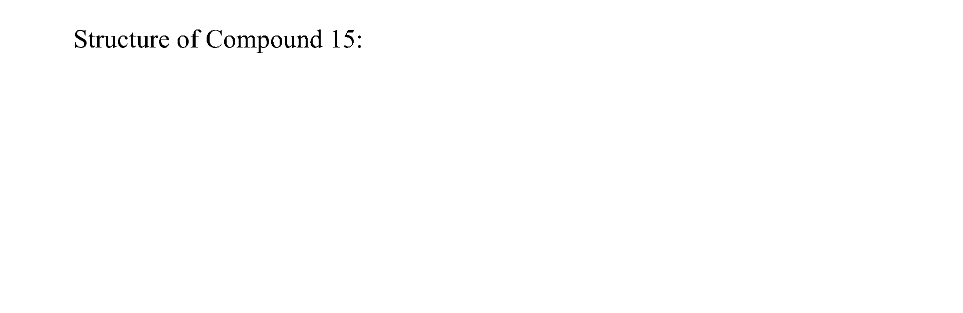

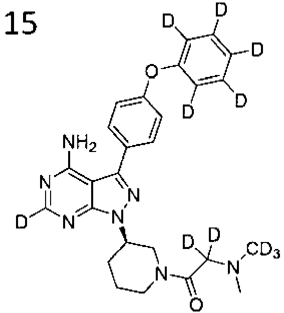

The study found that among the above compounds, Compounds 2 and 3 have a unique significant inhibitory effect on human acute monocytic leukemia cell strain MV4-11 and human acute myeloid leukemia cell strain MOLM-13. Furthermore, Compounds 2 and 3 exhibit superior pharmacokinetic properties compared to D18, which can significantly reduce the dosage and decrease toxic side effects.

Experimental instruments and reagents used in the present invention

Experimental instruments: Bruker AVIII 400MHz NMR spectrometer; Bruker AV NEO 600MHz NMR spectrometer; NICOLET Nexus 470FT-IR infrared spectrometer; Waters ACQUITY Arc high-performance liquid chromatograph; QDA (ESI) mass detector; Agilent G7890-5975C (EI) gas chromatography-mass spectrometer; Thermo Scientific LTQ Orbitrap XL highresolution mass spectrometer.

Reagents: Compound C₂₇H₃₀N₆O₃, ethyl acetate, hydrogen chloride dioxane, sodium bicarbonate, hydrogen chloride, 2-dimethylaminoacetic acid, HATU, DIPEA, EtOAc, dichloromethane, chloroacetyl chloride, MeOH, deuterated dimethylamine d₆ hydrochloride, triethylamine, potassium iodide, anhydrous sodium sulfate, petroleum ether, absolute ethanol, acetonitrile, etc. All reagents were commercially available analytical grade products and used directly after purchase.

### Synthesis of Compound D18

According to the following reaction scheme, a starting material (1 g, 2.06 mmol) was weighed and dissolved in ethyl acetate (20 mL). 4N hydrogen chloride dioxane solution was added under an ice-water bath. Then the reaction occured at room temperature for 2 hours. TLC test indicated complete reaction. The solvent was removed by reduced pressure distillation to obtain a white solid. Ethyl acetate and water were added. A 2N sodium bicarbonate solution was added. Extraction was performed with ethyl acetate. The combined ethyl acetate layers were washed with saturated brine, dried, and the solvent was removed by reduced pressure distillation to obtain 865 mg of a white solid.

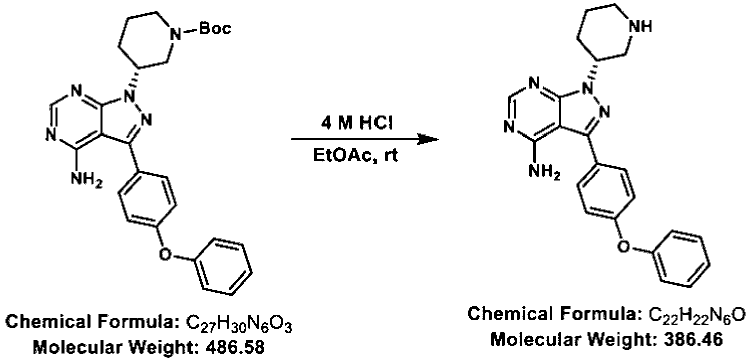

According to the following reaction scheme, 2-dimethylaminoacetic acid (22 mg, 0.21 mmol), HATU (80 mg, 0.21 mmol), and DIPEA (35 µL, 0.20 mmol) were added to a solution of the starting material (80 mg, 0.21 mmol) in DMF (4 mL). The reaction mixture was stirred for 5 h. Then it was diluted with EtOAc (50 mL), washed with water (20 mL × 3) and saturated brine (30 mL). The organic layer was dried over anhydrous sodium sulfate, concentrated, and purified by silica gel column chromatography (0-10% MeOH in DCM) to obtain a white solid Compound D-18 (67 mg, 68%). ¹H NMR (400 MHz, DMSO-d₆): δ_{H} 8.26 (d,J = 8.2 Hz, 1H), 7.71-7.61 (m, 2H), 7.43 (t,J = 7.8 Hz, 2H), 7.24-7.08 (m, 5H), 4.83 (m, 0.5H), 4.66 (s, 0.5H), 4.46 (dd,J = 12.6, 4.3 Hz, 1H), 4.24-4.08 (m, 1H), 3.93 (d,J = 13.4 Hz, 1H), 3.65 (s, 0.5H), 3.56-3.05 (m, 3H), 2.92 (s, 0.5H), 2.36 (s, 4H), 2.27 (s, 4H), 2.12 (d,J = 4.3 Hz, 1H), 1.90 (m, 1H), 1.69 (s, 1H). ¹³C NMR (100 MHz, DMSO-d₆) δ_{C} 166.7, 158.2, 157.1, 156.3, 155.7, 153.9, 143.3, 130.1, 127.9, 123.8, 120.1, 119.0, 97.4, 60.7, 52.6, 52.0, 49.0, 45.4, 44.9, 44.8, 41.4, 29.4, 24.5.

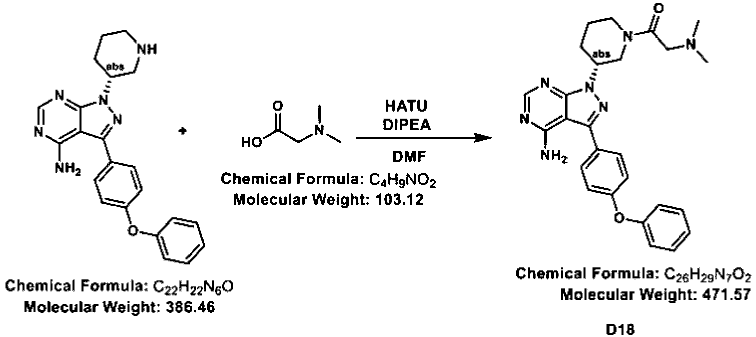

### Synthesis of Compound 2 (D18-D6)

According to the following reaction scheme, at 0°C, DIPEA (36.5 µL, 0.21 mmol) and chloroacetyl chloride (17 µL, 0.21 mmol) were added to a solution of the starting material (80 mg, 0.21 mmol) in dichloromethane (10 mL). The resulting mixture was stirred for 3 minutes. Then it was quenched with MeOH (5 mL), concentrated, and purified by silica gel column chromatography (dichloromethane:methanol 100:1 to 30:1) to obtain an amide compound (89 mg) as a white solid.

The above white solid was dissolved in dry acetonitrile (5 mL), and deuterated dimethylamine d₆ hydrochloride (88 mg, 1 mmol), triethylamine (140 µL, 2 mmol), and potassium iodide (17 mg, 0.1 mmol) were added. The temperature was raised to 40°C to react for 12 hours. Then water was added to quench the reaction. Extraction was performed with ethyl acetate three times. After drying, purification was performed by silica gel column chromatography (dichloromethane:methanol 50:1 to 10:1) to obtain a product D-18-d₆ as a white solid, 75 mg, total yield 75%. ¹H NMR (400 MHz, DMSO-d₆): δ_{H} 8.28 (d,J = 8.3 Hz, 1H), 7.68 (d,J = 8.2 Hz, 2H), 7.49-7.39 (m, 2H), 7.16 (m, 5H), 4.83 (m, 0.5H), 4.66 (m, 0.5H), 4.49 (dd,J = 12.5, 4.2 Hz, 1H), 4.19 (m, 1H), 4.10-3.95 (m, 1H), 3.63 (dd,J = 13.2, 9.7 Hz, 1H), 3.35-2.81 (m, 4H), 2.36-2.05 (m, 2H), 1.91 (m, 1H), 1.77-1.42 (m, 1H). ¹³C NMR (100 MHz, DMSO-d₆): δ_{C} 167.6, 158.2, 157.1, 156.3, 155.6, 153.9, 143.3, 130.1, 127.9, 123.8, 118.9, 97.5, 61.4, 53.1, 52.1, 49.3, 45.4, 45.4, 45.1, 41.7, 29.5, 24.7.

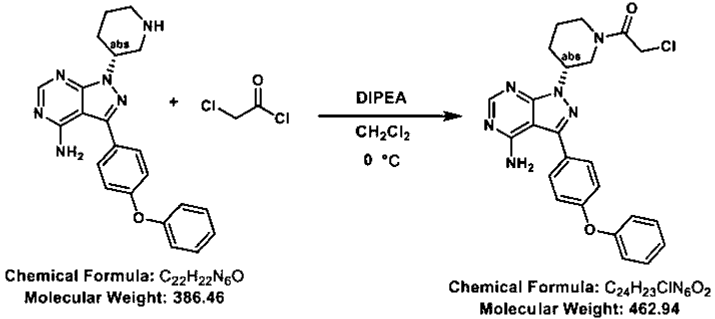

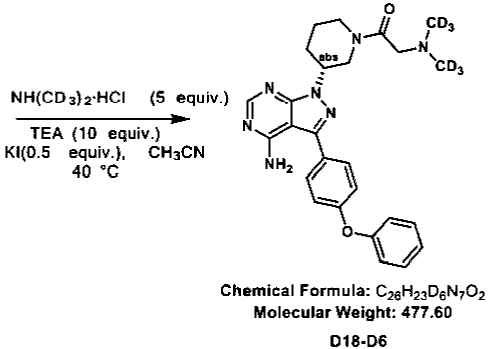

### Synthesis of Compound 3 (D18-D3)

According to the following reaction scheme, at 0°C, DIPEA (36.5 µL, 0.21 mmol) and chloroacetyl chloride (17 µL, 0.21 mmol) were added to a solution of the starting material (80 mg, 0.21 mmol) in dichloromethane (10 mL). The resulting mixture was stirred for 3 minutes. Then it was quenched with MeOH (5 mL), concentrated, and purified by silica gel column chromatography (dichloromethane:methanol 100:1 to 30:1) to obtain an amide compound (89 mg) as a white solid. The above solid was dissolved in dry acetonitrile (5 mL), and deuterated dimethylamine d₃ hydrochloride (88 mg, 1 mmol), triethylamine (140 µL, 2 mmol), and potassium iodide (17 mg, 0.1 mmol) were added. The temperature was raised to 40°C to react for 12 hours. Then water was added to quench the reaction. Extraction was performed with ethyl acetate three times. After drying, purification was performed by silica gel column chromatography (dichloromethane:methanol 50:1 to 10:1) to obtain a product D-18-d₃ as a white solid, 71 mg, total yield 70%.

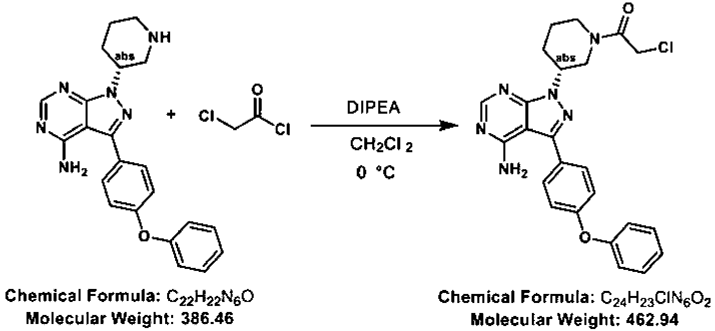

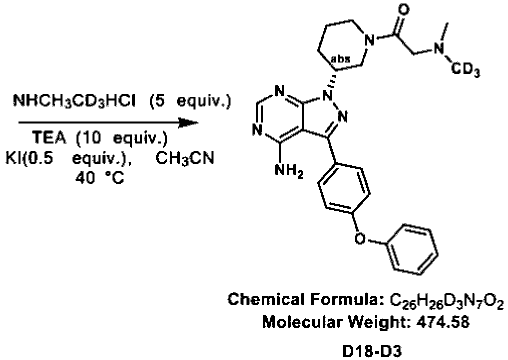

### Effect of D18 and deuterated series drugs on cancer cell proliferation

The inhibitory effect on cancer cell proliferation was assessed by measuring the effect of D18 and deuterated series drugs on cancer cell proliferation. Human acute monocytic leukemia cell strain MV4-11 (expressing FLT3/ITD mutant gene) and human acute myeloid leukemia cell strain MOLM-13 (expressing FLT3/ITD mutant gene and wild-type FLT3 gene) were selected. Different concentrations of the above compounds (25 nM, 50 nM, 100 nM, 200 nM, 400 nM in DMSO) were added to the above cells and incubated for 72 hours. The Cell Counting Kit-8 (CCK-8) reagent was used to detect the absorbance at 450 nm using a microplate reader to obtain OD values, and IC50 was further calculated. The experimental results are shown.

**Table 1 IC₅₀ values of compounds against MOLM-13 and MV4-11 cells**

| Compound | MV-411 IC₅₀ [µM] | MOLM-13 IC₅₀ [ µM] |
|---|---|---|
| D18 | 0.062 | 0.081 |
| Compound 2 (D18-D6) | 0.027 | 0.031 |
| Compound 3 (D18-D) | 0.042 | 0.048 |
| Compound 4 | 0.61 | 0.52 |
| Compound 5 | 0.53 | 0.57 |
| Compound 6 | 0.451 | 0.56 |
| Compound 7 | 0.49 | 0.45 |
| Compound 8 | 0.45 | 0.54 |
| Compound 9 | 0.56 | 0.45 |
| Compound 10 | 0.46 | 0.61 |
| Compound 11 | 0.73 | 0.85 |
| Compound 12 | 1.2 | 1.3 |
| Compound 13 | 1.45 | 1.23 |
| Compound 14 | 1.25 | 1.34 |
| Compound 15 | 1.25 | 1.45 |

The results show that among the series of deuterated compounds, Compounds 2 and 3 exhibit a unique inhibitory effect on cancer cell proliferation. The IC₅₀ values of Compounds 2 and 3 against MOLM-13 and MV4-11 cells are significantly lower than those of other deuterated compounds, and also considerably lower than those of Compound D18, indicating that their inhibitory effect on MOLM-13 and MV4-11 cells is not only significantly superior to other deuterated compounds but also considerably superior to compound D18. (IC₅₀, half maximal inhibitory concentration, refers to the concentration of a drug that induces 50% apoptosis in tumor cells, *i.e.,* the drug concentration corresponding to a ratio of apoptotic cells to total cells equal to 50%. The IC₅₀ value can be used to measure the ability of a drug to induce tumor cell apoptosis; a lower value indicates stronger inducing ability.)

### Effect of D18 and D18-D6 on cell cycle in cells

The effects of D18 and D18-D6 on the cell cycle distribution of human acute monocytic leukemia cell strain MV4-11 and acute myeloid leukemia cell strain MOLM-13 were tested. The two compounds at different concentrations (100 nM, 200 nM, 300 nM, 400 nM in DMSO) were applied to acute myeloid leukemia cell MV4-11 and MOLM-13 cell strain carrying the FLT3/ITD mutation gene. After 24 hours of treatment, cells were collected, washed twice with 1X PBS buffer solution, fixed with 75% ethanol at -20°C for 24 hours, and washed twice again with 1X PBS buffer. 0.5 mL 1X PBS buffer and 0.5 mL PI staining solution (purchased from BD Bioscience, USA) were added to the cells, and the cells were placed in the dark at 37°C for 15 minutes for staining. Cell cycle distribution was detected using a flow cytometer (BD FACS Calibur). Data were analyzed using FlowJo software (Ashland, OR).

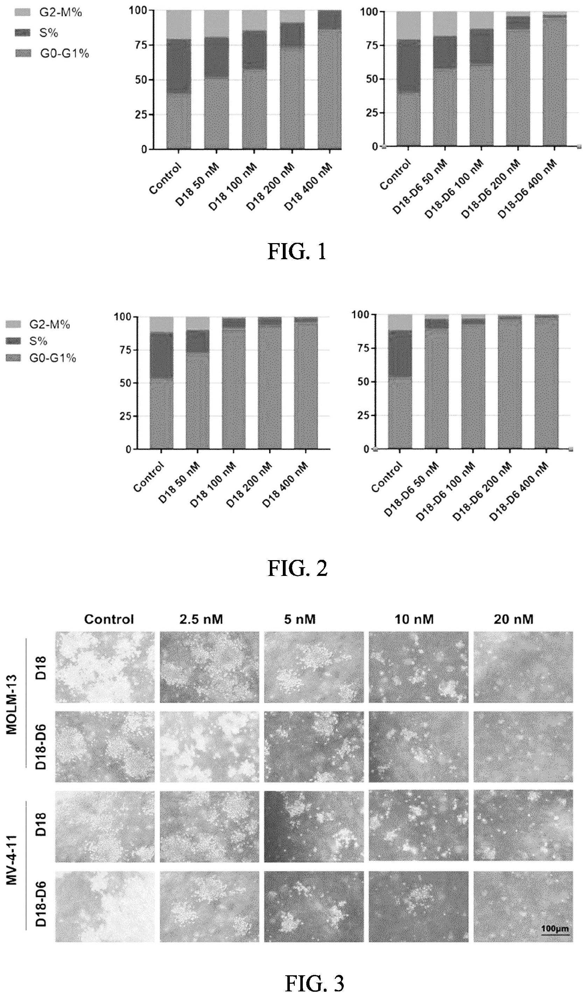

The experimental results are shown in FIGs. 1 and 2. Under the action of D18 and D18-D6, the cell cycle of MOLM-13 cells (FIG. 1) and MV4-11 cells (FIG. 2) was arrested in the G0-G1 phase in a dose-dependent manner.

### Effect of D18 and D18-D6 on cell clone formation

The proliferation ability of MOLM-13 and MV-4-11 cells carrying the FLT3/ITD mutation gene was assessed by soft agar colony formation assay. The two compounds at different concentrations (2.5 nM, 5 nM, 10 nM, 20 nM in DMSO) were applied to MOLM-13 and MV-4-11 cell strains carrying the FLT3/ITD mutation gene. The time required for sufficient colony formation was typically 15 days, with medium changed twice a week.

The experimental results are shown in FIG. 3. When the drug dosage was increased, the clones formed by MOLM-13 and MV4-11 cells were significantly reduced in size. Moreover, the number of colonies in the D18-D6 drug group was markedly reduced, indicating that D18-D6 has a stronger ability to attenuate the proliferation ability of AML cells compared to D18.

### Treatment of acute myeloid leukemia with D18 and D18-D6

To assess the tumor inhibitory effect in vivo, a nude mouse subcutaneous tumor-bearing model was introduced. More than 30 five-week-old mice (Balb/c-nu male mice, purchased from Hangzhou Ziyuan Experimental Animal Technology Co., Ltd.) were subcutaneously inoculated with MOLM-13 cells at 1×10⁷ cells per mouse. The body weight changes and tumor volume (tumor volume = tumor length × tumor width² / 2) of the mice were recorded daily. After 10 days, when the mouse tumor volume reached 200-400 mm³, the mice were randomly divided into 3 groups: vehicle group, D18 50 mg/kg dose group, and D18-D6 50 mg/kg dose group, with 6-10 mice per group. The following treatments were administered: Group 1 received daily oral gavage of vehicle (a liquid mixture formulation of 5% DMSO, 40% PEG-400, and 55% physiological saline); Group 2 received daily oral gavage of D18 liquid mixture formulation at 50 mg/kg; Group 3 received daily oral gavage of D18-D6 liquid mixture formulation at 50 mg/kg. The day of first administration was recorded as day 0, and administration was continued daily for 2 to 3 weeks of observation.

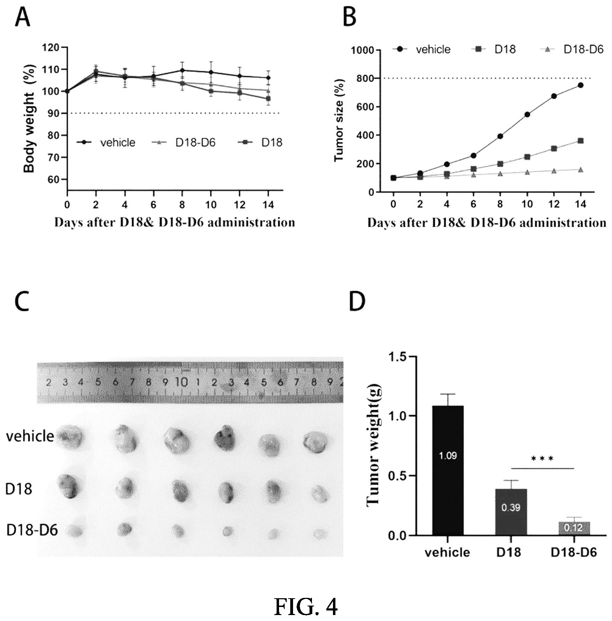

The experimental results are shown in FIG. 4. FIG. 4A shows the change in mouse body weight after administration; FIG. 4B shows the change in tumor size in mice after administration; FIG. 4C shows actual photos of final tumors in mice after administration; and FIG. 4D shows the weight of final tumors in mice after administration. The results show that after treating mice with D18, the mouse tumor growth was significantly inhibited, and the increase in mouse tumor volume was significantly slowed. After treating mice with D18-D6, the mouse tumor growth was strongly inhibited. In the D18 group, the increase in mouse tumor volume was significantly slowed, while in the D18-D6 group, the mouse tumors hardly grew. Data from the tumor xenograft mouse model indicate that both D18 and D18-D6 can inhibit the growth of acute myeloid leukemia (AML) tumors in mice, and the inhibitory effect of D18-D6 is significantly stronger than that of D18.

### Pharmacokinetic differences between D18 and D18-D6

The present invention also evaluated the pharmacokinetic (PK) properties of D18 and D18-D6 in rats following intravenous and oral administration. The results are shown in Tables 2 and 3. D18 had a half-life of approximately 1.5 h (iv) and 3.6 h (po), with a bioavailability of approximately 35.93%; D18-D6 had a half-life of approximately 1.9 h (iv) and 4.3 h (po), with a bioavailability of approximately 42.51%, indicating that the pharmacokinetic properties of D18-D6 are significantly improved compared to D18.

**Table 2 Pharmacokinetic properties of D18**

| | T_{1/2} (h) | Tₘₐₓ(h) | Cₘₐₓ (µg/L) | AUC₍₀₋₁₎ (µg/L•h) | AUC_{(0-∝)} (µg/L•h) | Vz (L/kg) | CLz (L/h/kg) | MRT_{(0-∝)} (h) | F(%) |
|---|---|---|---|---|---|---|---|---|---|
| iv(tmg/kg), mean | 1.537 | 0.017 | 767.665 | 684.615 | 695.331 | 3.304 | 1.488 | 1.49 | NA |
| SD | 0.307 | 0 | 201.181 | 120.443 | 120.982 | 0.982 | 0.342 | 0.273 | NA |
| po(10mg/kg), mean | 3.646 | 3 | 304.121 | 2465.816 | 2498.188 | 21.50 | 4.127 | 5.646 | 35.93 |
| SD | 1.006 | 1.095 | 77.174 | 407.461 | 419.29 | 6.052 | 0.897 | 0.819 | NA |

**Table 3 Pharmacokinetic properties of D18-D6**

| | T_{1/2} (h) | Tₘₐₓ(h) | Cₘₐₓ (µg/L) | AUC_{(0-∞)} (µg/L•h) | AUC_{(0-∞)} (µg/L•h) | Vz (L/kg) | CLz (L/h/kg) | MRT_{(0-∞)} (h) | F(%) |
|---|---|---|---|---|---|---|---|---|---|
| iv(1mg/kg), mean | 1.898 | 0.136 | 422.341 | 478.508 | 502.948 | 5.661 | 2.02 | 2.393 | NA |
| SD | 0.524 | 0.201 | 264.063 | 85.351 | 70.643 | 2.268 | 0.269 | 1.309 | NA |
| po(10mg/kg). mean | 4.333 | 2.583 | 184.886 | 2062.891 | 2137.891 | 30.65 | 4.997 | 5.957 | 42.51 |
| SD | 2.112 | 1.625 | 52.252 | 572.918 | 583.716 | 13.71 | 1.434 | 0.41 | NA |

The term "deuterated/deuteration " as used in the present invention refers to the replacement of one or more hydrogens in a compound or group with deuterium. Deuteration can be monosubstitution, disubstitution, polysubstitution, or persubstitution. After deuteration, the deuterium isotope abundance at the deuteriumsubstituted position is greater than the natural deuterium isotope abundance (0.015%), more preferably greater than 50%, more preferably greater than 75%, more preferably greater than 95%, more preferably greater than 97%, more preferably greater than 99%, more preferably greater than 99.5%.

The term "pharmaceutically acceptable salt" as used in the present invention refers to a salt formed by the compound of the present invention with an acid or base that is suitable for use as a drug. Pharmaceutically acceptable salts can include both inorganic salts and organic salts.

The term "active ingredient" as used in the present invention refers to any substance or mixture of substances used in the manufacture of a drug that has pharmacological activity or other direct effects in the diagnosis, treatment, symptom alleviation, management, or prevention of a disease, or can affect the function or structure of the body.

The term "pharmaceutically acceptable adjuvant" as used in the present invention has certain physiological activity, but the addition of this component does not change the dominant role of the above pharmaceutical composition in the disease treatment process, and merely exerts auxiliary effects. These auxiliary effects are merely the utilization of the known activity of this component and are conventional adjuvant treatment methods in the pharmaceutical field. If the above auxiliary components are used in combination with the pharmaceutical composition of the present invention, it should still fall within the protection scope of the present invention.

The above embodiments are exemplary, intended to illustrate the technical concept and features of the present invention, enabling those skilled in the art to understand the content of the present invention and implement it accordingly, and shall not be construed as limiting the protection scope of the present invention. Any equivalent changes or modifications made according to the spirit and essence of the present invention should be covered by the protection scope of the present invention.

## Claims

1. A compound or a pharmaceutically acceptable salt thereof, **characterized in that** the compound has a structure of formula (I):

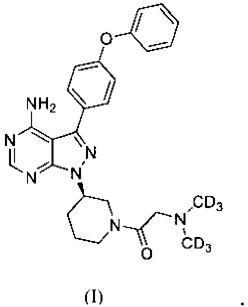

2. A compound or a pharmaceutically acceptable salt thereof, **characterized in that** the compound has a structure of formula (II):

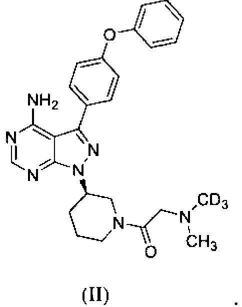

3. The compound or pharmaceutically acceptable salt thereof according to claim 1 or 2, **characterized in that** the pharmaceutically acceptable salt is a salt formed from the compound with an acid.

4. The compound or pharmaceutically acceptable salt thereof according to claim 1 or 2, **characterized in that** the pharmaceutically acceptable salt is selected from one or more of phosphate, camphorsulfonate, hydrochloride, hydrobromide, hydrofluoride, sulfate, nitrate, formate, acetate, propionate, oxalate, malonate, succinate, fumarate, maleate, lactate, malate, tartrate, citrate, picrate, methanesulfonate, trifluoromethanesulfonate, benzenemethanesulfonate, and benzene sulfonate.

5. The compound or pharmaceutically acceptable salt thereof according to claim 1 or 2, **characterized in that** the pharmaceutically acceptable salt is hydrochloride.

6. Use of the compound or pharmaceutically acceptable salt thereof according to any one of claims 1 to 5, **characterized in that** the compound or pharmaceutically acceptable salt thereof is used in the preparation of a drug for treating cancer.

7. The use of the compound or pharmaceutically acceptable salt thereof according to claim 6, **characterized in that** the cancer is leukemia.

8. The use of the compound or pharmaceutically acceptable salt thereof according to claim 6, **characterized in that** the cancer is one or more of lung cancer, colorectal cancer, pancreatic cancer, ovarian cancer, liver cancer, glioblastoma, solid tumor, non-small cell lung cancer, papillary renal cell carcinoma, and melanoma.

9. Use of the compound or pharmaceutically acceptable salt thereof according to any one of claims 1 to 5, **characterized in that** the compound or pharmaceutically acceptable salt thereof is used in the preparation of an FLT3 kinase inhibitor.

10. A drug for treating cancer, **characterized in that** the drug is prepared using the compound or pharmaceutically acceptable salt thereof according to any one of claims 1 to 5 as an active ingredient, together with pharmaceutically acceptable adjuvants.
